(19) Europäisches Patentamt
European Patent Office
Office européen des brevets

(11) **EP 2 643 496 B1**

(12) **FASCICULE DE BREVET EUROPEEN**

(45) Date de publication et mention
de la délivrance du brevet:
**12.04.2017 Bulletin 2017/15**

(21) Numéro de dépôt: **11788437.9**

(22) Date de dépôt: **24.11.2011**

(51) Int Cl.:
*C23C 18/12* [(2006.01)]  *G01N 21/64* [(2006.01)]
*B01J 20/10* [(2006.01)]  *C01B 37/00* [(2006.01)]
*C01B 37/02* [(2006.01)]  *C12Q 1/00* [(2006.01)]
*G01N 31/22* [(2006.01)]  *G01N 33/22* [(2006.01)]
*C01B 33/141* [(2006.01)]

(86) Numéro de dépôt international:
**PCT/EP2011/070903**

(87) Numéro de publication internationale:
**WO 2012/069569 (31.05.2012 Gazette 2012/22)**

(54) **UTILISATION DE FILMS MINCES DE SILICES MESOPOREUSES COMME MATERIAUX SENSIBLES DANS DES CAPTEURS GRAVIMETRIQUES POUR LA DETECTION OU LE DOSAGE DE VAPEURS DE COMPOSES NITRES**

VERWENDUNG VON MESOPORÖSEN SILICA DÜNNFILMEN ALS SENSITIVES ELEMENT IN GRAVIMETRISCHEN SENSOREN ZUR BESTIMMUNG VON NITRIERTEN VERBINDUNGEN IN GASEN

USE OF THIN FILMS OF MESOPOROUS SILICA AS SENSITIVE COMPONENTS IN GRAVIMETRIC SENSORS FOR DETERMINING THE CONCENTRATION OF NITRATE COMPOUNDS IN GASES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.11.2010 FR 1059776**

(43) Date de publication de la demande:
**02.10.2013 Bulletin 2013/40**

(73) Titulaire: **Commissariat à l'Énergie Atomique et aux Énergies Alternatives
75015 Paris (FR)**

(72) Inventeurs:
• **MONTMEAT, Pierre**
**F-37520 La Riche (FR)**
• **HAIRAULT, Lionel**
**F-37150 Bléré (FR)**
• **BELLEVILLE, Philippe**
**F-37000 Tours (FR)**
• **PRENE, Philippe**
**F-75003 Paris (FR)**
• **THERY-MERLAND, Frédéric**
**F-37360 Neuillé-Pont-Pierre (FR)**
• **LE GUEVEL, Xavier**
**F-44410 Herbignac (FR)**

(74) Mandataire: **Ilgart, Jean-Christophe
BREVALEX
95, rue d'Amsterdam
75378 Paris Cedex 8 (FR)**

(56) Documents cités:
**WO-A1-02/16914     FR-A1- 2 929 283**

• **BELLEVILLE ET AL: "Functional coatings: The sol-gel approach", COMPTES RENDUS - CHIMIE, ELSEVIER, PARIS, FR, vol. 13, no. 1-2, 1 janvier 2010 (2010-01-01), pages 97-105, XP026940148, ISSN: 1631-0748, DOI: 10.1016/J.CRCI.2009.12.005 [extrait le 2010-02-09]**
• **SHENGYANG TAO ET AL: "Porphyrin-doped mesoporous silica films for rapid TNT detection", COLLOID AND POLYMER SCIENCE ; KOLLOID-ZEITSCHRIFT UND ZEITSCHRIFT FÜR POLYMERE, SPRINGER, BERLIN, DE, vol. 285, no. 7, 10 février 2007 (2007-02-10), pages 721-728, XP019516489, ISSN: 1435-1536, DOI: DOI:10.1007/S00396-007-1643-7**

EP 2 643 496 B1

**Description**

**DOMAINE TECHNIQUE**

La présente invention se rapporte à l'utilisation de films minces de silices mésoporeuses obtenues par un procédé sol-gel en tant que matériaux sensibles dans des capteurs chimiques et, plus précisément, gravimétriques destinés à détecter ou à doser des composés nitrés sous forme de vapeurs, et en particulier des composés nitroaromatiques tels que le nitrobenzène (NB), le dinitrobenzène (DNB), le trinitrobenzène (TNB), le nitrotoluène (NT), le dinitrotoluène (DNT), le 2,4,6-trinitrotoluène (TNT) et analogues.

De tels capteurs sont notamment utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant, stockant et/ou manipulant des composés nitrés.

**ETAT DE LA TECHNIQUE ANTERIEURE**

A l'heure actuelle, plusieurs méthodes sont utilisées pour détecter des vapeurs de composés nitrés comme l'emploi de chiens "*renifleurs*" dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité mais ne donnent toutefois pas totalement satisfaction.

Ainsi, l'utilisation de chiens "*renifleurs*" présente l'inconvénient de nécessiter une longue formation des chiens et de leurs maîtres et d'être inadaptée à des opérations de détection prolongées en raison de la durée limitée que présente l'attention des chiens.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique est modifiée au contact des molécules gazeuses recherchées, qui revêt un système apte à mesurer en temps réel toute variation de cette propriété physique et de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

Pour la détection de composés nitrés gazeux, et plus particulièrement de composés nitroaromatiques, des études ont été menées sur différentes familles de matériaux, à savoir des polysiloxanes fonctionnalisés, des polyéthylèneglycols, des composés organiques cycliques tels qu'une phtalocyanine de cuivre ou une cyclodextrine, des dendrimères de polyphénylène, des adsorbants du type charbon actif et des composés fluorescents.

Il est, par ailleurs, connu que des silices mésoporeuses élaborées par un procédé sol-gel, et en particulier des silices mésoporeuses de type MTS (Mésoporeux aux Tensioactifs Structurants), sont susceptibles d'être utilisées dans des capteurs chimiques.

Toutefois, il est généralement admis que, pour que des silices mésoporeuses puissent permettre une détection d'une famille de composés qui soit à la fois sensible et spécifique à ces composés, il convient que soient présentes dans leurs pores des molécules sondes adaptées aux composés devant être détectés, c'est-à-dire des molécules qui sont capables d'interagir sélectivement avec eux et d'en révéler ainsi la présence au sein d'un mélange complexe par l'émission d'un signal détectable.

De fait, les seules études qui semblent avoir été réalisées sur la détection de composés nitrés au moyen de capteurs munis d'un film mince de silice mésoporeuse concernent une détection par fluorescence rendue possible par l'incorporation, dans la silice mésoporeuse et au cours de sa fabrication, d'une porphyrine (Shengyang et al., Colloid Polym. Sci. 2007, 285, 721-728, **[1],** Shengyang et al., ChemPhysChem 2006, 7, 1902-1905, **[2]).**

Or, dans le cadre de leurs travaux, les Inventeurs ont constaté que, contrairement à ce qui est généralement décrit et admis dans la littérature, des capteurs comprenant des films minces de silices mésoporeuses obtenues par un procédé sol-gel permettent une détection très sensible et spécifique des composés nitrés sous forme de vapeurs et ce, sans qu'il soit nécessaire d'incorporer dans les pores de ces silices des molécules sonde adaptées à ce type de composés.

Ils ont, en outre, constaté que les performances de détection de ces capteurs restent intactes pendant au moins une année.

## EXPOSÉ DE L'INVENTION

La présente invention a donc pour objet l'utilisation d'un film mince de silice mésoporeuse <u>ayant été</u> obtenue par un procédé sol-gel en tant que matériau sensible dans un capteur gravimétrique pour la détection ou le dosage de vapeurs d'un ou plusieurs composés <u>nitrés, dans laquelle la silice est fonctionnalisée par des groupes méthyles.</u>

Dans ce qui précède et ce qui suit, on entend par *"silice mésoporeuse"*, une silice poreuse dont la taille des pores est comprise entre 2 et 50 nanomètres, conformément aux recommandations pour la caractérisa-tion de solides poreux de l'INTERNATIONAL UNION OF PURE AND APPLIED CHEMISTRY (Rouquérol et al., Pure & Applied Chemistry, 66(8), 1994, pages 1739-1758, **[3]**).

On entend, par ailleurs, par *"composé nitré"*, tout composé ayant au moins un groupe nitro, c'est-à-dire un groupe -NO$_2$, conformément à la définition donnée par l'INTERNATIONAL UNION OF PURE AND APPLIED CHEMISTRY dans son Compendium de Terminologie Chimique.

La technique sol-gel étant bien connue de l'homme du métier, nous rappellerons simplement que cette technique consiste en une polymérisation minérale et qu'elle comprend une réaction d'hydrolyse d'un ou plusieurs précurseurs d'oxyde dans une phase solvant pour former des fonctions hydroxyles réactives, suivie d'une réaction de condensation pour former des ponts "oxo" par élimination de molécules d'eau ou d'alcool.

Ainsi, par exemple, dans le cas de précurseurs organiques d'oxyde de silicium, ces réactions s'écrivent schématiquement :

<u>Hydrolyse</u> : Si-OR + H$_2$O → Si-OH + R-OH

<u>Condensation</u> : Si-OH + RO-Si → Si-O-Si + R-OH
Si-OH + HO-Si → Si-O-Si + H$_2$O.

Elles conduisent tout d'abord à la formation d'une solution qui est appelée *"sol"* mais qui peut être, selon les conditions dans lesquelles est réalisée l'hydrolyse des précurseurs d'oxyde, soit un sol au sens strict du terme (c'est-à-dire une solution renfermant des espèces chimiques sous forme de colloïdes), soit une solution renfermant des espèces chimiques sous forme d'oligomères ou de polymères, puis à la formation d'un *"gel"* (c'est-à-dire une masse visqueuse, élastique et présentant une structure de liquide figé) qui est constitué d'un réseau polymérique minéral et dont la viscosité augmente avec le temps.

Après élimination de la phase solvant emprisonnée dans le gel, ce dernier peut être soumis à des traitements complémentaires comme, par exemple, un traitement thermique permettant de le densifier.

Dans le cadre de la présente invention, le film mince de silice mésoporeuse mesure, avantageusement, de 1 nanomètre à 1 micromètre d'épaisseur.

Il est, par ailleurs, de préférence obtenu par dépôt d'une ou plusieurs couches d'un sol de silice colloïdale sur au moins l'une des faces d'un substrat et élimination de la phase solvant présente dans cette couche ou ces couches.

Ce sol, qui renferme typiquement des colloïdes d'oxyde de silicium présentant un diamètre de l'ordre de 1 nanomètre à 100 micromètres, est, de préférence, préparé par hydrolyse basique d'au moins un précurseur d'oxyde de silicium dans une phase solvant contenant un solvant organique, seul ou mélangé à de l'eau, puis mûrissement de la solution ainsi obtenue.

Dans ce cas, le sol de silice colloïdale est tout préférentiellement préparé par un procédé qui comprend les étapes suivantes :

a) hydrolyse d'au moins un précurseur d'oxyde de silicium dans un alcool ou un mélange d'alcools additionné d'une base forte et présentant un pH au moins égal à 8,
b) mûrissement de la solution ainsi obtenue pour former un sol alcoolique de silice colloïdale, et éventuellement
c) élimination de la base forte du sol alcoolique ainsi formé ou remplacement par de l'eau de tout ou partie de l'alcool ou des alcools présents dans ce sol.

Ainsi, le sol de silice colloïdale peut aussi bien être un sol alcoolique, un sol hydroalcoolique qu'un sol aqueux.

L'élimination de la base forte du sol obtenu à l'étape b) ci-avant peut être réalisée en traitant ce dernier au reflux par un solvant organique, par exemple un alcool ou un mélange d'alcools.

Quant au remplacement par de l'eau de tout ou partie de l'alcool ou des alcools présents dans le sol obtenu à l'étape b) ci-avant peut être réalisé par dilution de ce sol par un mélange d'eau et d'un ou plusieurs alcools pour obtenir un sol hydroalcoolique, puis par concentration du sol hydroalcoolique ainsi obtenu dans des conditions qui permettent d'éliminer tout ou partie de l'alcool ou des alcools qu'il renferme.

En variante, le sol de silice colloïdale peut également être préparé par hydrolyse acide d'au moins un précurseur d'oxyde de silicium en solution dans une phase solvant contenant un solvant organique, seul ou mélangé à de l'eau, et dont le pH est, de préférence, compris entre 0 et 1, puis mélange de la solution ainsi obtenue avec une solution contenant de l'eau, un solvant organique et au moins un agent tensioactif, et mûrissement du mélange résultant.

Dans ce cas, la réaction de condensation nécessaire à la formation des ponts "oxo", qui, pour une silice mésoporeuse, sont des ponts siloxanes, s'effectue autour des micelles que forme l'agent tensioactif en solution et conduit à l'obtention d'une phase hybride organique-inorganique pouvant présenter éventuellement une structure périodiquement organisée à plus ou moins longue distance.

L'élimination de cet agent tensioactif, une fois le film mince de silice mésoporeuse constitué, par un lavage au moyen d'un solvant ou par un traitement thermique, permet d'obtenir une silice mésoporeuse dotée d'une structure éventuellement périodique à plus ou moins longue distance, par exemple de type cubique, hexagonale, lamellaire ou vermiculaire, et qui est dite de ce fait *"mésostructurée"*.

L'agent tensioactif est choisi en fonction du type de mésostructure que l'on souhaite conférer à la silice, de la taille des pores et des murs de cette mésostructure ainsi que de la possibilité de solubiliser cet agent tensioactif dans la solution contenant le précurseur d'oxyde de silicium.

Ainsi, par exemple, on utilise, de préférence, un agent tensioactif amphiphile à chaîne courte tel qu'un sel d'alkyltriméthylammonium pour obtenir une silice mésostructurée dont la taille des pores ne dépasse pas 10 nanomètres et dont la taille des murs est de l'ordre de 1 nanomètre, tandis que l'on utilise, de préférence, un agent tensioactif constitué d'un copolymère multiblocs amphiphile, comprenant au moins un bloc hydrophobe associé à au moins un bloc hydrophile, tels que ceux commercialisés sous la dénomination Pluronic® à base d'oxyde de polyéthylène (EO) et d'oxyde de polypropylène (PO) de type $(EO)_n$-$(PO)_m$-$(EO)_n$ pour obtenir une silice mésostructurée présentant une plus grande taille de pores (jusqu'à 50 nanomètres).

Quelle que soit la voie d'hydrolyse retenue, le précurseur d'oxyde de silicium est, de préférence, choisi parmi les alcoxydes de silicium tétrafonctionnels de formule $Si(OR)_4$ dans laquelle R représente un groupe alkyle comprenant de 1 à 6 atomes de carbone et, notamment, parmi le tétraméthylorthosilicate (TMOS) et le tétraéthylorthosilicate (TEOS).

Toutefois, d'autres types de précurseur d'oxyde sont également susceptibles d'être utilisés comme, par exemple :

* des alcoxydes tétrafonctionnels de formule $Si(OR_4)$ dans laquelle R est un groupe autre qu'un groupe alkyle, tel qu'un groupe acétyle ;
* des alcoxydes de silicium mono-, di- ou trifonctionnels de formule $Si(OR)_{4-x}R'_x$ dans laquelle R est un groupe alkyle en $C_1$-$C_6$, tandis que R' est un groupe organique ou inorganique, par exemple un atome de chlore, et x est égal à 1, 2 ou 3 ; ou encore
* des silicates inorganiques tels que $SiCl_4$, $SiBr_4$ ou $Si_3O_7Na_2$.

Le solvant organique est, lui, de préférence, un alcool ou un mélange d'alcools, en particulier un alcool aliphatique ou un mélange d'alcools aliphatiques comprenant de 1 à 6 atomes de carbone tels que le méthanol, l'éthanol ou l'iso-propanol.

Toutefois, d'autres types de solvant organique peuvent aussi être utilisés comme, par exemple, des phénols ou des diols de formule OH-R"-OH dans laquelle R" est un groupe aldéhyde en $C_2$-$C_{30}$ ou un groupe phényle.

Par ailleurs, le sol de silice colloïdale présente avantageusement une teneur massique en silice allant de 1 à 10% et, de préférence, de 2 à 6%.

Le dépôt de la couche ou des couches du sol de silice colloïdale sur le substrat peut être effectué par l'une des techniques de dépôt par voie humide suivantes :

* la pulvérisation (connue sous la terminologie anglaise "*spray-coating*") ;
* l'enduction centrifuge (connue sous la terminologie anglaise "*spin-coating*") ;
* le dépôt à la goutte (connu sous la terminologie anglaise "*drop-coating*" *;*
* le trempage-retrait (connu sous la terminologie anglaise "*dip-coating*") ;
* l'enduction laminaire (connue sous la terminologie anglaise "*meniscus-coating*") ;
* l'épandage (connu sous la terminologie anglaise *"soak-coating"*) ;
* l'enduction au rouleau (connue sous la terminologie anglaise "*roll to roll process*") ; ou encore
* l'enduction au pinceau (connue sous la terminologie anglaise "*paint-coating*")*.

Parmi ces techniques, qui sont bien connues de l'homme du métier, on préfère la pulvérisation, l'enduction centrifuge, le trempage-retrait et le dépôt à la goutte car ce sont celles qui conviennent le mieux à la réalisation d'un film mince par dépôt d'un sol de silice colloïdale sur un substrat.

Quelle que soit la technique de dépôt utilisée, la phase solvant présente dans la couche ou les couches déposées sur le substrat est, de préférence, éliminée par évaporation, cette dernière pouvant se faire naturellement à l'air libre ou pouvant être facilitée, par exemple par l'application d'un flux gazeux, par chauffage thermique ou radiatif (UV, IR ou micro-ondes) pour autant que ce chauffage n'altère pas le substrat sous-jacent, ou encore par des moyens mécaniques comme lors d'un dépôt du sol de silice colloïdale sur le substrat par enduction centrifuge.

Conformément à l'invention, la silice peut être fonctionnalisée par des groupes chimiques susceptibles d'améliorer les performances du capteur gravimétrique, par exemple en renforçant sa sensibilité et/ou sa sélectivité vis-à-vis des composés nitrés, en augmentant sa réversibilité et/ou sa durabilité, ou encore en diminuant sa sensibilité aux variations hygrométriques environnementales.

Ainsi, la sensibilité et la sélectivité du capteur gravimétrique vis-à-vis des composés nitrés peuvent être renforcées par un greffage de groupes chimiques présentant une forte affinité pour ce type de composés, auquel cas ce greffage est, par exemple, réalisé en faisant réagir des groupes hydroxyles libres de la silice avec un composé de formule $X_{(4-x-y-z)}\text{-}SiR^1{}_xR^2{}_yR^3{}_z$ dans laquelle X est un groupe hydrolysable comme un halogénure, un acétonate, un acrylate ou un alcoolate de formule $OR^4$ où $R^4$ est un groupe alkyle comprenant de 1 à 10 atomes de carbone, $R^1$, $R^2$ et $R^3$, qui peuvent être identiques ou différents, sont des groupes sensibles aux composés nitrés, par exemple du type aniline, pyrène ou dansyle, x, y et z sont des nombres entiers allant de 0 à 3 à la condition toutefois que l'un au moins de x, y et Z soit différent de 0.

La sensibilité du capteur gravimétrique aux variations hygrométriques environnementales peut, elle, être diminuée par un greffage de groupes hydrophobes et, notamment, de groupes alkyles, de groupes aromatiques (phényles par exemple) ou de groupes fluorés comme, par exemple, des chaînes alkyles comportant plusieurs atomes de fluor du type $-CH_2\text{-}CH_2\text{-}(CF_2)_7\text{-}CF_3$, auquel cas ce greffage est, par exemple, réalisé en faisant réagir des groupes hydroxyles libres de la silice avec un composé de formule $X_{(4-x-y-z)}\text{-}SiR^1{}_xR^2{}_yR^3{}_z$ dans laquelle X, x, y et z ont la même signification que précédemment, mais dans laquelle $R^1$, $R^2$ et $R^3$ sont des groupes hydrophobes. Il peut également être réalisé en faisant réagir des groupes hydroxyles libres de la silice avec un composé de formule $R^4\text{-}Si\text{-}NH\text{-}Si\text{-}R^5$ dans laquelle $R^4$ et $R^5$, qui peuvent être identiques ou différents, sont des groupes hydrophobes.

A cet égard, la méthylation de la silice, que l'on réalise en utilisant, par exemple, de l'hexaméthyldisilazane, s'est révélée être particulièrement avantageuse car elle permet de conserver au film mince de silice mésoporeuse un niveau de sensibilité aux composés nitrés très satisfaisant sur une large gamme de degrés d'hygrométrie (0-80%), à température ambiante.

Quels que soient les groupes chimiques que l'on entend greffer sur la silice, le greffage de ces groupes peut être réalisé soit sur la silice à l'état de sol colloïdal, avant que celui-ci ne soit déposé sur le substrat, soit sur la silice à l'état de film mince, une fois celui-ci constitué sur le substrat.

Dans ce dernier cas, le greffage est effectué en mettant ce film mince en contact avec un composé comprenant au moins un groupe correspondant à ceux que l'on souhaite greffer, soit sous forme vapeur si les composés que l'on souhaite greffer sont volatils, soit sous forme liquide s'ils ne le sont pas.

Néanmoins, pour une fonctionnalisation de la silice par des groupes méthyles, on préfère soumettre le film mince de silice mésoporeuse à une méthylation de surface, par exemple par traitement de ce film par des vapeurs d'hexaméthyldisilazane, alors que, pour une fonctionnalisation de la silice par des groupes fluorophores, on fait réagir la silice avec un composé comprenant un ou plusieurs groupes fluorophores aussi bien lorsqu'elle est à l'état de sol colloïdal que sous forme de film mince, notamment en trempant ce film dans une solution de ce composé.

Par ailleurs, indépendamment de toute fonctionnalisation de la silice, le film mince de silice mésoporeuse peut être soumis à un ou plusieurs traitements choisis parmi :

* un lavage par un solvant organique afin d'éliminer l'agent tensioactif présent dans ce film et d'obtenir ainsi une silice mésostructurée lorsqu'un agent de ce type a été utilisé au cours de la préparation du sol de silice colloïdale ;
* une densification par voie thermique ou radiative (UV, IR ou micro-ondes) ; ou encore
* un durcissement par voie chimique.

La densification par voie thermique, qui consiste à chauffer le film mince de silice mésoporeuse à une température élevée, c'est-à-dire pouvant aller jusqu'à 900°C, mais toutefois inférieure à la température d'endommagement du substrat, permet de consolider ce film mince et, dans le cas où un agent tensioactif a été utilisé lors de la préparation du sol de silice colloïdale, d'éliminer cet agent pour obtenir une silice mésostructurée.

Le durcissement par voie chimique, qui est décrit dans FR-A-2 703 791 **[4],** consiste à faire subir au film mince de silice mésoporeuse un traitement alcalin en milieu liquide ou gazeux, typiquement en présence de molécules d'ammoniac, ou un traitement acide. Il permet d'améliorer, non seulement la tenue mécanique de ce film mince et, en particulier, sa résistance à l'abrasion et son adhésion sur le substrat, mais également les performances de détection du capteur gravimétrique.

Conformément à l'invention, le capteur est un capteur gravimétrique.

A titre d'exemples de capteur gravimétrique, on peut citer les capteurs à microbalance à quartz, les capteurs à ondes de surface (connus sous la terminologie anglaise "SAW" pour "*Surface Acoustic Wave*") tels que les capteurs à ondes de Love et les capteurs à ondes de Lamb, et les microleviers.

Parmi les capteurs gravimétriques, on préfère plus particulièrement les capteurs du type microbalance à quartz. Ce type de capteur, dont le principe de fonctionnement a été décrit par Sanchez-Pedrono et al. (Anal. Chem. Acta, 182, 1986, page 285, [5]), comprend, schématiquement, un substrat piézo-électrique (ou résonateur), généralement un cristal de quartz recouvert sur ses deux faces d'une couche métallique, par exemple d'or ou de platine, et qui est relié à deux électrodes. Le matériau sensible recouvrant l'une ou les deux faces du substrat, toute variation de masse de ce matériau se traduit par une variation de la fréquence de vibration du substrat.

Le capteur gravimétrique peut également être un multicapteur, c'est-à-dire qu'il peut comprendre plusieurs capteurs élémentaires qui sont munis de matériaux sensibles et/ou de substrats différents les uns des autres, l'essentiel étant que l'un au moins de ces capteurs élémentaires comprenne un film mince d'une silice mésoporeuse tel que précédemment décrit, en tant que matériau sensible.

Conformément à l'invention, le ou les composés nitrés destinés à être détectés par le capteur gravimétrique sont, de préférence, des composés nitroaromatiques, des nitramines, des nitrosamines et/ou des esters nitriques.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'amino-dinitrotoluène, le dinitrotrifluorométhylbenzène, le chlorodinitrotrifluorométhylbenzène, l'hexanitro-stilbène, la trinitrophénylméthylnitramine (ou tétryle) ou encore le trinitrophénol (ou acide picrique).

Les nitramines sont, elles, par exemple la cyclotétraméthylènetétranitramine (ou octogène), la cyclotriméthylènetrinitramine (ou hexogène) et le tétryl, tandis que les nitrosamines sont, par exemple, la nitrosodiméthylamine.

Quant aux esters nitriques, il s'agit, par exemple, de pentrite, de dinitrate d'éthylène glycol, de dinitrate de diéthylène glycol, de nitroglycérine ou de nitroguanidine.

L'utilisation d'une silice mésoporeuse obtenue par un procédé sol-gel sous la forme d'un film mince en tant que matériau sensible dans un capteur gravimétrique pour la détection de composés nitrés sous forme de vapeurs s'est révélée présenter de nombreux avantages.

En effet, non seulement des capteurs munis d'un tel matériau sensible sont capables de détecter spécifiquement des vapeurs de composés nitrés, et en particulier des vapeurs de composés nitroaromatiques, avec une très grande sensibilité - puisqu'ils peuvent détecter leur présence à des concentrations dans l'air de l'ordre du ppm (partie par million) et même inférieures -, ils se caractérisent par :

* une rapidité de réponse et une reproductibilité de cette réponse ;
* une sélectivité vis-à-vis des composés nitrés;
* une stabilité de leurs performances dans le temps et une durée de vie très satisfaisante ;
* une stabilité de leurs performances dans une large gamme d'hygrométrie ambiante ;
* une aptitude à fonctionner en continu ;
* une simplicité de fabrication liée au fait que, d'une part, le procédé sol-gel est un procédé simple à mettre en oeuvre et que, d'autre part, il n'est pas nécessaire d'incorporer des molécules sonde spécifiques aux composés nitrés dans les pores des films minces de silice ;
* un coût compatible avec une production de capteurs en série ; et
* la possibilité d'être miniaturisés et, ainsi, d'être aisément transportables et manipulables sur tout type de site.

D'autres caractéristiques et avantages de l'invention apparaîtront mieux à la lecture du complément de description qui suit, qui se rapporte à des exemples illustrant la préparation de sols propres à être utilisés pour réaliser des films minces de silice mésoporeuse, l'utilisation pour la détection de vapeurs de composés nitroaromatiques de capteurs à microbalance à quartz comprenant des films minces de silice mésoporeuse obtenus à partir de ces sols, ainsi qu'à des exemples illustrant l'influence d'un durcissement à l'ammoniac et d'une méthylation de surface de ces films sur les performances de détection des capteurs.

Bien entendu, les exemples qui suivent ne sont donnés qu'à titre d'illustrations de l'objet de l'invention et ne constituent en aucun cas une limitation de cet objet.

## BREVE DESCRIPTION DES FIGURES

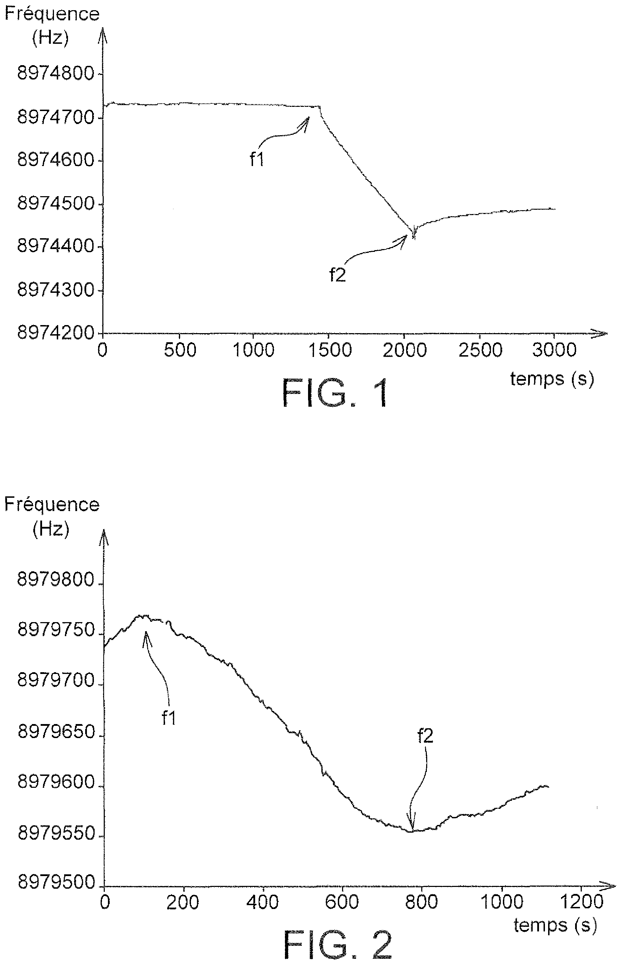

La figure 1 représente l'évolution de la fréquence de vibration du quartz telle qu'observée pour un capteur à microbalance à quartz comprenant un film mince de silice mésoporeuse lorsque ce capteur est respectivement exposé à l'air ambiant et à des vapeurs de 2,4-dinitrotrifluorométhoxybenzène (DNTFMB).

La figure 2 représente l'évolution de la fréquence de vibration du quartz telle qu'observée pour un capteur à microbalance à quartz comprenant un film mince de silice mésoporeuse lorsque ce capteur est respectivement exposé à l'air ambiant et à des vapeurs de dinitrofluorobenzène (DNFB).

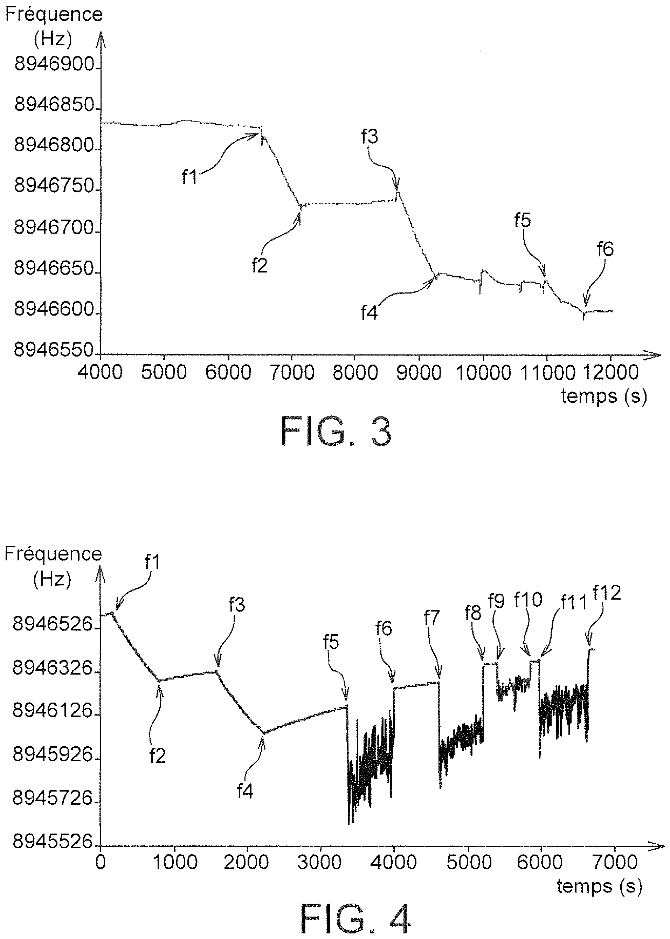

La figure 3 représente l'évolution de la fréquence de vibration du quartz telle qu'observée pour un capteur à microbalance à quartz comprenant un film mince de silice mésoporeuse lorsque ce capteur est respectivement exposé à l'air ambiant, à des vapeurs de 2,4-dinitrotoluène (2,4-DNT) et à des vapeurs de 1,3-dinitrobenzène (1,3-DNB).

La figure 4 représente l'évolution de la fréquence de vibration du quartz telle qu'observée pour un capteur à microbalance à quartz comprenant un film mince de silice mésoporeuse lorsque ce capteur est respectivement exposé à l'air ambiant, à des vapeurs de DNTFMB, à des vapeurs de dichlorométhane, à des vapeurs de méthyléthylcétone, à des vapeurs de toluène et à des vapeurs d'éthanol.

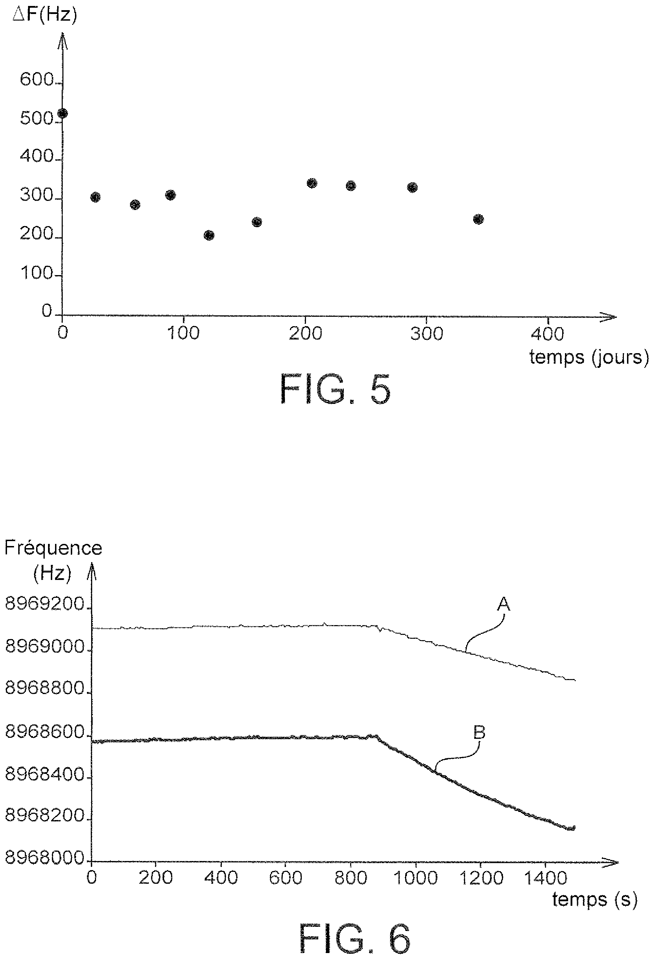

La figure 5 représente les variations de la fréquence de vibration du quartz d'un capteur à microbalance à quartz comprenant un film mince de silice mésoporeuse, telles qu'observées lorsque ce capteur est soumis à dix expositions à des vapeurs de DNTFMB de 10 minutes chacune, réparties sur une période de 350 jours.

La figure 6 représente l'évolution de la fréquence de vibration du quartz telle qu'observée pour un capteur à microbalance à quartz comprenant un film mince de silice mésoporeuse lorsque ce capteur est respectivement exposé à l'air ambiant et à des vapeurs de DNTFMB, avant et après avoir soumis ledit film mince à un traitement de durcissement à l'ammoniac.

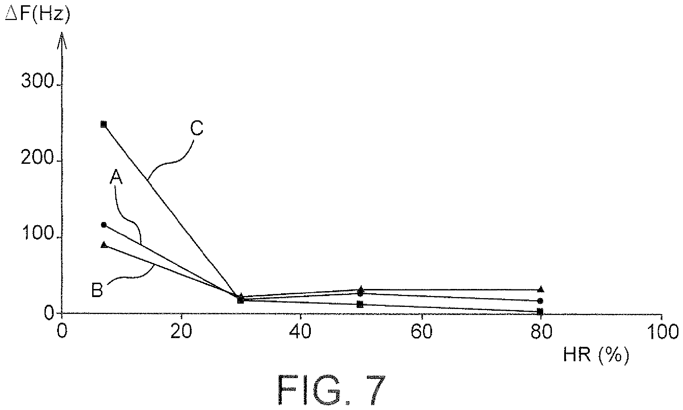

La figure 7 représente les variations de la fréquence de vibration du quartz telles qu'observées pour trois capteurs à microbalance à quartz, dont deux comprennent un film mince d'une silice mésoporeuse ayant été soumis à un traitement de méthylation et dont l'un comprend un film mince de silice mésoporeuse n'ayant pas été soumis à ce traitement, lorsque ces capteurs sont exposés à des vapeurs de DNTFMB dans de l'air présentant différents degrés d'hygrométrie.

## EXEMPLES

### Exemple 1 : Préparation d'un sol éthanolique de silice colloïdale

On prépare tout d'abord un sol de silice colloïdale en milieu éthanolique basique, puis on ramène secondairement le pH de ce sol à une valeur de l'ordre de 6.

Pour ce faire, on mélange 1000 g d'éthanol absolu à 130,7 g de tétraéthylorthosilicate (TEOS) de formule $Si(OCH_2CH_3)_4$ pendant 10 minutes sous agitation magnétique. On ajoute alors 34,7 g d'ammoniaque à 28% en masse, toujours sous agitation magnétique. On poursuit l'agitation pendant 2 heures après quoi on laisse mûrir la solution ainsi obtenue, dont le pH est de l'ordre de 10, à 25°C pendant une semaine.

Au terme de la semaine de mûrissement, on élimine l'ammoniaque présent dans le sol de silice colloïdale ainsi formé en mettant ce dernier au reflux à 78°C pendant 24 heures. On obtient alors un sol éthanolique de silice colloïdale au pH d'environ 6 et dont la teneur massique en silice colloïdale est de 3,4%.

Avant utilisation, ce sol est filtré sur un filtre Whatmann® en polypropylène de 0,45 micromètres.

### Exemple 2 : Préparation d'un sol hydroéthanolique de silice colloïdale

A un sol de silice colloïdale en milieu éthanolique basique tel que préparé dans l'exemple 1 ci-avant, on ajoute un mélange d'eau et d'éthanol absolu dans un rapport massique eau/éthanol de 50/50, sous agitation magnétique.

Après 1 heure d'agitation, la solution est concentrée sous pression réduite (70 mPa) à 40°C jusqu'à ce que la teneur massique soit ramenée à celle du sol éthanolique basique de départ, c'est-à-dire à une valeur de 3,4% environ, ce qui conduit à l'obtention d'un sol présentant une teneur massique en eau de l'ordre de 40%.

Avant utilisation, ce sol est filtré sur un filtre Whatmann® en polypropylène de 0,45 micromètres.

### Exemple 3 : Détection de vapeurs de DNTFMB par un capteur à microbalance à quartz comprenant un film mince de silice mésoporeuse

On recouvre les deux faces d'un quartz de coupe AT, de fréquence de vibration de 9 MHz et muni de deux électrodes de mesure circulaires en or (modèle QA9RA-50, AMETEK PRECISION INSTRUMENTS), d'un film mince de silice mésoporeuse en déposant sur chacune de ces faces une couche d'un sol hydroéthanolique de silice colloïdale tel que préparé dans l'exemple 2 ci-avant, par enduction centrifuge à 2000 tours/minute et en maintenant la rotation du substrat à cette vitesse pendant 5 minutes pour éliminer de cette couche l'eau et l'alcool qu'elle renferme par séchage à température ambiante et sous pression atmosphérique.

La variation de la fréquence du quartz liée à la présence du film mince de silice mésoporeuse est de 8 kHz.

Le capteur ainsi obtenu est exposé successivement à :

- l'air ambiant pendant 25 minutes,
- du DNTFMB à une concentration de 3 ppm dans l'air ambiant pendant 10 minutes, et
- l'air ambiant pendant 15 minutes,

la température de l'air et du DNTFMB étant de 25°C.

La figure 1 illustre l'évolution de la fréquence de vibration du quartz observée au cours de ces expositions sous la forme d'une courbe représentant les valeurs de la fréquence de vibration du quartz, exprimées en hertz (Hz), en fonction du temps, exprimé en secondes (s), les flèches f1 et f2 signalant respectivement le début et la fin de l'exposition au DNTFMB.

Elle montre que l'exposition du capteur au DNTFMB provoque une nette diminution de la fréquence de vibration du quartz de ce capteur (257 Hz pour 10 minutes d'exposition).

**Exemple 4 : Détection du DNFB par un capteur à microbalance à quartz comprenant un film mince de silice mésoporeuse**

On recouvre les deux faces d'un quartz identique à celui utilisé dans l'exemple 3 précédent, d'un film mince de silice mésoporeuse en déposant sur chacune de ces faces une couche d'un sol éthanolique de silice colloïdale tel que préparé dans l'exemple 1 précédent, par enduction centrifuge à 2000 tours/ minute et en maintenant la rotation du substrat à cette vitesse pendant 5 minutes pour éliminer de cette couche l'éthanol qu'elle renferme par séchage à température ambiante et sous pression atmosphérique.

La variation de la fréquence du quartz liée à la présence du film mince de silice mésoporeuse est de 10 kHz.

Le capteur ainsi obtenu est exposé successivement à :

- l'air ambiant pendant 2 minutes,
- du DNFB à une concentration de 800 ppb dans l'air ambiant pendant 10 minutes, et
- l'air ambiant pendant 5 minutes,

la température de l'air et du DNFB étant de 25°C.

La figure 2 illustre l'évolution de la fréquence de vibration du quartz observée au cours de ces expositions sous la forme d'une courbe représentant les valeurs de la fréquence de vibration du quartz, exprimées en hertz (Hz), en fonction du temps, exprimé en secondes (s), les flèches f1 et f2 signalant respectivement le début et la fin de l'exposition au DNFB.

Elle montre que l'exposition du capteur au DNFB provoque une nette diminution de la fréquence de vibration du quartz de ce capteur (196 Hz pour 10 minutes d'exposition).

**Exemple 5 : Détection du 2,4-DNT et du 1,3-DNB par un capteur à microbalance à quartz comprenant un film mince de silice mésoporeuse**

Dans cet exemple, on utilise un capteur identique à celui utilisé dans l'exemple 4 précédent.

Le capteur est exposé successivement à :

- l'air ambiant pendant 40 minutes,
- du 2,4-DNT à une concentration de 285 ppb dans l'air ambiant pendant 10 minutes,
- l'air ambiant pendant 20 minutes,
- du 2,4-DNT à une concentration de 285 ppb dans l'air ambiant pendant 10 minutes,
- l'air ambiant pendant 30 minutes,
- du 1,3-DNB à une concentration de 5 ppm dans l'air ambiant pendant 10 minutes, et
- l'air ambiant pendant 5 minutes,

la température de l'air, du 2,4-DNT et du 1,3-DNB étant de 25°C.

La figure 3 illustre l'évolution de la fréquence de vibration du quartz observée au cours de ces expositions sous la forme d'une courbe représentant les valeurs de la fréquence de vibration du quartz, exprimées en hertz (Hz), en fonction du temps, exprimé en secondes (s). Sur cette courbe, les flèches f1 et f2 indiquent respectivement le début et la fin de la première exposition au 2,4-DNT ; les flèches f3 et f4 indiquent respectivement le début et la fin de la seconde exposition au 2,4-DNT tandis que les flèches f5 et f6 indiquent respectivement le début et la fin de l'exposition au 1,3-DNB.

Comme le montre cette figure, on observe, lorsque le capteur est mis en présence de 2,4-DNT ou de 1,3-DNB, une nette diminution de la fréquence de vibration du quartz.

**Exemple 6 : Mise en évidence de la sélectivité vis-à-vis des composés nitrés d'un capteur à microbalance à quartz comprenant un film mince de silice mésoporeuse**

Dans cet exemple, on utilise un capteur identique à celui utilisé dans les exemples 4 et 5 précédents.

On expose ce capteur successivement à :

- l'air ambiant pendant 3 minutes,
- du DNTFMB à une concentration de 3 ppm dans l'air ambiant pendant 10 minutes,
- l'air ambiant pendant 9 minutes,
- du DNTFMB à une concentration de 3 ppm dans l'air ambiant pendant 10 minutes,
- l'air ambiant pendant 15 minutes,
- du dichlorométhane à une concentration de 580 000 ppm dans l'air ambiant pendant 10 minutes,
- l'air ambiant pendant 8 minutes,
- de la méthyléthylcétone à une concentration de 126 000 ppm dans l'air ambiant pendant 10 minutes,
- l'air ambiant pendant 3 minutes,
- du toluène à une concentration de 38 000 ppm dans l'air ambiant pendant 7 minutes,
- l'air ambiant pendant 3 minutes,
- de l'éthanol à une concentration de 79 000 ppm dans l'air ambiant pendant 10 minutes, et
- l'air ambiant pendant 2 minutes,

la température de l'air, du DNTFMB et des différents solvants organiques étant de 25°C.

La figure 4 illustre l'évolution de la fréquence de vibration du quartz observée au cours de ces expositions sous la forme d'une courbe représentant les valeurs de la fréquence de vibration du quartz, exprimées en hertz (Hz), en fonction du temps, exprimé en secondes (s).

Sur cette courbe, les flèches f1 et f2 indiquent respectivement le début et la fin de la première exposition au DNTFMB ; les flèches f3 et f4 indiquent respectivement le début et la fin de la seconde exposition au DNTFMB ; les flèches f5 et f6 indiquent respectivement le début et la fin de l'exposition au dichlorométhane ; les flèches f7 et f8 indiquent respectivement le début et la fin de l'exposition à la méthyléthylcétone ; les flèches f9 et f10 indiquent respectivement le début et la fin de l'exposition au toluène, tandis que les flèches f11 et f12 indiquent respectivement le début et la fin de l'exposition à l'éthanol.

Comme le montre la figure 4, on assiste, lorsque le capteur est exposé à du DNTFMB ou à des solvants organiques, à une nette diminution de la fréquence du quartz de ce capteur.

Cette diminution atteint respectivement 286 Hz et 246 Hz lors des première et seconde expositions de 10 minutes au DNTFMB. Les variations de fréquence obtenues dans le cas des solvants organiques sont du même ordre de grandeur que celles observées pour le DNTFMB. Toutefois, en comparaison à la très faible concentration en DNTFMB utilisée (3 ppm), les très fortes concentrations en solvants testées (supérieures à 38 000 ppm) indiquent que l'on est en présence d'un capteur faisant preuve d'une très grande sensibilité et d'une très grande spécificité à l'égard du DNTFMB en présence de solvants organiques.

**Exemple 7 : Mise en évidence de la stabilité dans le temps des performances d'un capteur à microbalance à quartz comprenant un film mince de silice mésoporeuse**

Dans cet exemple, on utilise un capteur identique à celui utilisé dans les exemples 4 à 6 précédents.

Le jour de sa fabrication, le capteur est exposé à du DNTFMB, à une concentration de 3 ppm dans l'air ambiant, pendant 10 minutes.

Puis, il est conservé à l'air ambiant et soumis à neuf autres expositions à du DNTFMB, toujours à une concentration égale à 3 ppm dans l'air ambiant et d'une durée de 10 minutes chacune, réparties sur une période de 350 jours.

La figure 5 représente les valeurs des variations de la fréquence de vibration ($\Delta F$) du quartz, exprimées en hertz (Hz), telles qu'obtenues pour ces dix expositions, ces valeurs étant déterminées pour chaque exposition comme suit :

$$\Delta F = \text{fréquence de vibration au temps } t_0 \text{ de l'exposition} - \text{fréquence de vibration au temps } t_{10min} \text{ de l'exposition,}$$

et symbolisées par des points noirs sur ladite figure 5.

Sur cette figure, le jour 0 correspond au jour de la fabrication du capteur.

Cette figure montre que la variation de fréquence du quartz observée est toujours supérieure à 200 Hz même 350 jours après la fabrication du capteur. On est donc en présence d'un capteur dont les performances sont stables dans le temps et qui est toujours capable de détecter du DNTFMB avec une très grande sensibilité pendant une période d'au moins un an après son élaboration et sans prendre de précaution particulière quant aux conditions de conservation de ce capteur.

### Exemple 8 : Influence d'un traitement de durcissement à l'ammoniac du film mince de silice mésoporeuse d'un capteur à microbalance à quartz sur les performances de détection de ce capteur

On recouvre les deux faces d'un quartz identique à ceux utilisés dans les exemples précédents d'une couche mince de silice mésoporeuse en déposant sur chacune de ces faces une couche d'un sol hydroéthanolique de silice colloïdale tel que préparé dans l'exemple 2 ci-avant, par enduction centrifuge à 2000 tours/minute et en maintenant la rotation du substrat à cette vitesse pendant 5 minutes pour éliminer de cette couche l'eau et l'éthanol qu'elle renferme par séchage à température ambiante et sous pression atmosphérique.

La variation de la fréquence du quartz liée à la présence du film mince de silice mésoporeuse est de 10 kHz.

Le capteur ainsi obtenu est exposé à l'air ambiant pendant 14 minutes, puis à du DNTFMB à une concentration de 3 ppm dans l'air ambiant pendant 10 minutes (la température de l'air et du DNTFMB étant de 25°C) et la fréquence de vibration du quartz est mesurée pendant toute la durée de ces expositions.

Le film mince de silice mésoporeuse recouvrant le quartz est ensuite soumis à un traitement de durcissement à l'ammoniac. Ce traitement consiste à placer le capteur sur un support dans une enceinte close d'un volume de 6 000 $cm^3$, contenant environ 500 $cm^3$ d'une solution d'ammoniac à 28%, et à le maintenir en confinement ammoniacal pendant une période de 12 heures minimum.

A l'issue de ce traitement, le capteur est de nouveau exposé à l'air ambiant pendant 14 minutes, puis à du DNTFMB à une concentration de 3 ppm dans l'air ambiant, pendant 10 minutes et dans les mêmes conditions que précédemment, et la fréquence de vibration du quartz est mesurée pendant toute la durée de ces expositions.

La figure 6 illustre l'évolution de la fréquence de vibration du quartz du capteur observée dans les deux séries d'expositions sous la forme de deux courbes A et B représentant les valeurs de la fréquence de vibration du quartz, exprimées en hertz (Hz), en fonction du temps, exprimé en secondes (s), la courbe A correspondant aux valeurs obtenues avant traitement du film mince de silice mésoporeuse et la courbe B correspondant aux valeurs obtenues après traitement de ce film mince.

Cette figure montre que ce traitement permet d'améliorer notablement les performances de détection du capteur puisque, pour une même durée d'exposition du capteur au DNTFMB (10 minutes), la fréquence de vibration du quartz diminue de 400 Hz après traitement alors qu'elle ne diminue que de 240 Hz avant traitement.

### Exemple 9 : Influence d'une méthylation de surface du film mince de silice mésoporeuse d'un capteur à microbalance à quartz sur les performances de détection de ce capteur

On prépare trois capteurs à microbalance à quartz comme décrit dans l'exemple 4 précédent et on soumet le film mince de silice mésoporeuse de deux de ces capteurs à une méthylation de surface par immersion pendant 12 heures dans une solution d'hexaméthyl-disilazane (HMDS) de formule $(CH_3)_3$-Si-NH-Si-$(CH_3)_3$ en faisant varier le rapport molaire HMDS/Si.

Puis, les trois capteurs sont soumis à une série d'expositions à des vapeurs de DNTFMB à une concentration de 0,1 ppm dans de l'air dont on fait varier le degré d'hygrométrie d'une exposition à l'autre. Le débit est fixé à 20 L/heure, le degré d'hygrométrie varie de 5 à 80% et les variations de fréquence des capteurs sont mesurées après 10 minutes d'exposition aux vapeurs de DNTFMB.

La figure 7 illustre les variations de la fréquence de vibration ($\Delta F$) du quartz, exprimées en hertz (Hz), telles qu'observées en fonction du degré d'hygrométrie (HR) de l'air, exprimé en %, pour les trois capteurs, la courbe A correspondant à un capteur dont la silice a été méthylée dans un rapport HMDS/Si de 0,2, la courbe B correspondant à au capteur dont la silice a été méthylée dans un rapport HMDS/Si de 1,5 et la courbe C correspondant au capteur dont la silice n'a pas été méthylée.

Cette figure montre que la sensibilité aux composés nitroaromatiques de capteurs comprenant un film mince de silice méthylée est peu affectée par le degré d'hygrométrie du milieu dans lequel se situent ces composés, contrairement à la sensibilité d'un capteur comprenant un film mince de silice non méthylée.

Une méthylation de surface du film mince de silice mésoporeuse permet donc d'atténuer les effets interférents de l'humidité environnementale.

**EP 2 643 496 B1**

**REFERENCES CITEES**

[1] Shengyang et al., Colloid Polym. Sci. 2007, 285, 721-728
[2] Shengyang et al., ChemPhysChem 2006, 7, 1902-1905
[3] Rouquérol et al., Pure & Applied Chemistry, 66(8), 1994, pages 1739-1758
[4] FR-A-2 703 791
[5] Pedrono et al., Anal. Chem. Acta, 182, 1986, page 285

**Revendications**

1. Utilisation d'un film mince de silice mésoporeuse ayant été obtenue par un procédé sol-gel en tant que matériau sensible dans un capteur gravimétrique pour la détection ou le dosage de vapeurs d'un ou plusieurs composés nitrés, dans laquelle la silice est fonctionnalisée par des groupes méthyles.

2. Utilisation selon la revendication 1, dans laquelle le film mince de silice mésoporeuse mesure de 1 nanomètre à 10 micromètres d'épaisseur.

3. Utilisation selon la revendication 1 ou la revendication 2, dans laquelle le film mince de silice mésoporeuse a été réalisé par dépôt d'une ou plusieurs couches d'un sol de silice colloïdale sur au moins l'une des faces d'un substrat et élimination de la phase solvant présente dans cette couche ou ces couches.

4. Utilisation selon la revendication 3, dans laquelle le sol de silice colloïdale renfermait des colloïdes d'oxyde de silicium présentant un diamètre de 1 nanomètre à 1 micromètre.

5. Utilisation selon l'une quelconque des revendications 3 et 4, dans laquelle le sol de silice colloïdale a été préparé par hydrolyse basique d'au moins un précurseur d'oxyde de silicium dans une phase solvant contenant un solvant organique, seul ou mélangé à de l'eau, puis mûrissement de la solution ainsi obtenue.

6. Utilisation selon la revendication 5, dans laquelle le sol de silice colloïdale a été préparé par un procédé qui comprend les étapes suivantes :

   a) hydrolyse d'au moins un précurseur d'oxyde de silicium dans un alcool ou un mélange d'alcools additionné d'une base forte et présentant un pH au moins égal à 8,
   b) mûrissement de la solution obtenue à l'étape a) pour obtenir un sol alcoolique de silice colloïdale, et éventuellement
   c) élimination de la base forte du sol obtenu à l'étape b) ou remplacement par de l'eau de tout ou partie de l'alcool ou des alcools présents dans ce sol.

7. Utilisation selon la revendication 6, dans laquelle le précurseur d'oxyde de silicium a été choisi parmi les alcoxydes de silicium de formule $Si(OR)_4$ dans laquelle R représente un groupe alkyle comprenant de 1 à 6 atomes de carbone.

8. Utilisation selon la revendication 7, dans laquelle le précurseur d'oxyde de silicium a été choisi parmi le tétraméthylorthosilicate (TMOS) et le tétraéthylorthosilicate (TEOS).

9. Utilisation selon la revendication 5, dans laquelle le solvant organique était un alcool ou un mélange d'alcools, de préférence un alcool aliphatique ou un mélange d'alcools aliphatiques comportant de 1 à 6 atomes de carbone.

10. Utilisation selon l'une quelconque des revendications 3 à 9, dans laquelle le sol de silice colloïdale présentait une teneur massique en silice allant de 1 à 10%, de préférence de 2 à 6%.

11. Utilisation selon l'une quelconque des revendications 3 à 10, dans laquelle le dépôt de la couche ou des couches du sol de silice colloïdale sur le substrat a été réalisé par pulvérisation, enduction centrifuge, dépôt à la goutte, trempage-retrait, enduction laminaire, épandage, enduction au rouleau ou encore enduction au pinceau.

12. Utilisation selon l'une quelconque des revendications 3 à 11, dans laquelle l'élimination de la phase solvant présente dans la couche ou les couches déposées sur le substrat a été réalisée par évaporation de cette phase.

**13.** Utilisation selon l'une quelconque des revendications 1 à 12, dans laquelle la fonctionnalisation de la silice par des groupes méthyles a été obtenue en soumettant le film mince de silice mésoporeuse à une méthylation de surface.

**14.** Utilisation selon l'une quelconque des revendications 1 à 13, dans laquelle le film mince de silice mésoporeuse a été soumis à au moins un traitement choisi parmi un lavage par un solvant organique, une densification par voie thermique et un durcissement par voie chimique.

**15.** Utilisation selon la revendication 14, dans laquelle le traitement de durcissement par voie chimique du film mince était un traitement par une base en milieu liquide ou gazeux, de préférence des vapeurs d'ammoniac.

**16.** Utilisation selon l'une quelconque des revendications 1 à 15, dans laquelle le capteur gravimétrique est un capteur à microbalance à quartz.

**17.** Utilisation selon l'une quelconque des revendications 1 à 16, dans laquelle le capteur gravimétrique est un multicapteur comprenant plusieurs capteurs élémentaires dont l'un au moins comprend un film mince de silice mésoporeuse en tant que matériau sensible.

**18.** Utilisation selon l'une quelconque des revendications 1 à 17, dans laquelle le ou les composés nitrés à détecter ou à doser sont choisis parmi les composés nitroaromatiques, les nitramines, les nitrosamines et les esters nitriques.

**19.** Utilisation selon l'une quelconque des revendications 1 à 18, dans laquelle le ou les composés nitrés à détecter ou à doser sont choisis parmi le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène, la trinitrophénylméthylnitramine et le trinitrophénol.

**Patentansprüche**

**1.** Verwendung eines mesoporösen Silika-Dünnfilms, der durch ein Sol-Gel-Verfahren erhalten wurde, als sensibles Material in einem gravimetrischem Sensor für die Erfassung oder die Dosierung von Dämpfen einer oder mehrerer nitrierten Verbindungen, wobei das Silika durch Methylgruppen funktionalisiert ist.

**2.** Verwendung nach Anspruch 1, wobei der mesoporöse Silika-Dünnfilm eine Dicke von 1 Nanometer bis 10 Mikrometer aufweist.

**3.** Verwendung nach Anspruch 1 oder Anspruch 2, wobei der mesoporöse Silika-Dünnfilm durch Aufbringen einer oder mehrerer Schichten eines kolloidalen Silika-Sols auf wenigstens einer der Flächen eines Substrats und Entfernen der Lösungsmittelphase realisiert worden ist, die in dieser Schicht oder diesen Schichten vorhanden ist.

**4.** Verwendung nach Anspruch 3, wobei das kolloidale Silika-Sol Siliciumoxyd-Kolloide einschloss, die einen Durchmesser von 1 Nanometer bis 1 Mikrometer aufweisen.

**5.** Verwendung nach einem der Ansprüche 3 und 4, wobei das kolloidale Silika-Sol hergestellt worden ist durch basische Hydrolyse wenigstens eines Präcursors von Siliciumoxyd in einer Lösungsmittelphase, die ein organisches Lösungsmittel alleine oder mit Wasser gemischt enthält und anschließendes Reifenlassen der derart erhaltenen Lösung.

**6.** Verwendung nach Anspruch 5, wobei das kolloidale Silika-Sol durch ein Verfahren hergestellt worden ist, das die folgenden Schritte umfasst:

a) Hydrolyse wenigstens eines Präcursors von Silicumoxyd in einem Alkohol oder einer Mischung von Alkoholen, zu denen eine starke Base hinzugefügt ist, und der/die einen pH von wenigstens gleich 8 aufweist,
b) Reifenlassen der im Schritt a) erhaltenen Lösung, um ein alkoholisches kolloidales Silika-Sol zu erhalten und gegebenenfalls
c) Entfernen der starken Base aus dem im Schritt b) erhaltenen Sol oder Ersetzen des gesamten oder eines Teils des Alkohols oder der Alkohole, die in diesem Sol vorhanden sind, durch Wasser.

7. Verwendung nach Anspruch 6, wobei der Präcursor von Siliciumoxyd ausgewählt wurde aus den Silicium-Alkoxyden der Formel Si(OR)$_4$, wobei R eine Alkylgruppe mit 1 bis 6 Kohlenstoffatomen repräsentiert.

8. Verwendung nach Anspruch 7, wobei der Präcursor von Siliciumoxyd ausgewählt wurde aus Tetramethylorthosilikat (TMOS) und Tetraethylorthosilikat (TEOS).

9. Verwendung nach Anspruch 5, wobei das organische Lösungsmittel ein Alkohol oder eine Mischung von Alkoholen war, vorzugsweise ein aliphatischer Alkohol oder eine Mischung von aliphatischen Alkoholen mit 1 bis 6 Kohlenstoffatomen.

10. Verwendung nach einem der Ansprüche 3 bis 9, wobei das kolloidale Silika-Sol einen Massengehalt an Silika von 1 bis 10% aufwies, vorzugsweise von 2 bis 6%.

11. Verwendung nach einem der Ansprüche 3 bis 10, wobei das Aufbringen der Schicht oder der Schichten des kolloidalen Silika-Sols auf das Substrat realisiert wurde durch Pulverisierung, Zentrifugalbeschichtung, Tropfenabscheidung, Tauchziehen, Laminarbeschichtung, Ausbringen, Rollenbeschichtung oder Streichbeschichtung.

12. Verwendung nach einem der Ansprüche 3 bis 11, wobei das Entfernen der Lösungsmittelphase, die in der Schicht oder den Schichten vorhanden war, die auf das Substrat aufgebracht wurden, durch Verdampfung dieser Phase realisiert wurde.

13. Verwendung nach einem der Ansprüche 1 bis 12, wobei die Funktionalisierung des Silika durch Methylgruppen erhalten wurde, indem der mesoporöse Silika-Dünnfilm einer Oberflächen-Methylierung unterzogen wurde.

14. Verwendung nach einem der Ansprüche 1 bis 13, wobei der mesoporöse Silika-Dünnfilm wenigstens einer Behandlung unterzogen wurde, ausgewählt aus einem Waschen mit einem organischen Lösungsmittel, einem Verdichten auf thermischen Wege und einem Härten auf chemischen Wege.

15. Verwendung nach Anspruch 14, wobei die Härtungsbehandlung des Dünnfilms auf chemischen Wege eine Behandlung mit einer Base in einer flüssigen oder gasförmigen Umgebung war, vorzugsweise mit Ammoniakdämpfen.

16. Verwendung nach einem der Ansprüche 1 bis 15, wobei der gravimetrische Sensor ein Quarzmikrowaagen Sensor ist.

17. Verwendung nach einem der Ansprüche 1 bis 16, wobei der gravimetrische Sensor ein Multisensor ist, der mehrere Elementarsensoren umfasst, von denen wenigstens einer einen mikropRösen Silika-Dünnfilm als sensibles Material umfasst.

18. Verwendung nach einem der Ansprüche 1 bis 17, wobei die zu erfassende(n) oder zu dosierende(n) nitrierte(n) Verbindung(en) ausgewählt ist/sind aus den nitroaromatischen Verbindungen, den Nitraminen, den Nitrosaminen und den Nitratestern.

19. Verwendung nach einem der Ansprüche 1 bis 18, wobei die zu erfassende(n) oder zu dosierende(n) Verbindung(en) ausgewählt ist/sind aus Nitrobenzol, Dinitrobenzol, Trinitrobenzol, Nitrotoluol, Dinitrotoluol, Trinitrotoluol, Dinitrofluorbenzol, Dinitrotrifluormethoxybenzol, Aminodinitrotoluol, Dinitrotriflourmethylbenzol, Chlorodinitrotrifluormethylbenzol, Hexanitrostilben, Trinitrophenylmethylnitramin und Trinitrophenol.

## Claims

1. Use of a thin film of mesoporous silica that has been obtained by a sol-gel method as a sensitive material in a gravimetric sensor for detecting or assaying vapours of one or more nitro compounds, wherein the silica is functionalised by methyl groups.

2. Use according to claim 1, wherein the thin film of mesoporous silica is of a thickness from 1 nanometre to 10 micrometres.

3. Use according to claim 1 or claim 2, wherein the thin film of mesoporous silica has been made by depositing one or more layers of a colloidal silica sol on at least one of the faces of a substrate and removing the solvent phase present in this layer or these layers.

4. Use according to claim 3, wherein the colloidal silica sol contained silicon oxide colloids having a diameter from 1 nanometre to 1 micrometre.

5. Use according to any of claims 3 and 4, wherein the colloidal silica sol has been prepared by basic hydrolysis of at least one silicon oxide precursor in a solvent phase containing an organic solvent, alone or mixed with water, and then ripening the solution thus obtained.

6. Use according to claim 5, wherein the colloidal silica sol has been prepared by a method which comprises the following steps:

   a) hydrolysing at least one silicon oxide precursor in an alcohol or an alcohol mixture added with a strong base and having a pH at least equal to 8,
   b) ripening the solution obtained in step a) to obtain an alcoholic sol of colloidal silica, and optionally
   c) removing the strong base from the sol obtained in step b) or replacing with water all or part of the alcohol or alcohols present in this sol.

7. Use according to claim 6, wherein the silicon oxide precursor has been chosen from silicon alkoxides of the formula $Si(OR)_4$ in which R represents an alkyl group comprising from 1 to 6 carbon atoms.

8. Use according to claim 7, wherein the silicon oxide precursor has been chosen from tetramethylorthosilicate (TMOS) and tetraethylorthosilicate (TEOS) .

9. Use according to claim 5, wherein the organic solvent was an alcohol or an alcohol mixture, preferably an aliphatic alcohol or an aliphatic alcohol mixture comprising from 1 to 6 carbon atoms.

10. Use according to any of claims 3 to 9, wherein the colloidal silica sol had a silica mass content ranging from 1 to 10%, preferably from 2 to 6%.

11. Use according to any of claims 3 to 10, wherein the deposition of the layer or layers of the colloidal silica sol onto the substrate has been made by spray-coating, spin-coating, drop-coating, dip-coating, meniscus-coating, soak-coating, roll to roll process or even paint-coating.

12. Use according to any of claims 3 to 11, wherein the removal of the solvent phase present in the layer or layers deposited onto the substrate has been made by evaporating this phase.

13. Use according to any of claims 1 to 12, wherein the functionalisation of the silica by methyl groups has been achieved by subjecting the thin film of mesoporous silica to a surface methylation.

14. Use according to any of claims 1 to 13, wherein the thin film of mesoporous silica has been subjected to at least one treatment chosen from an organic solvent washing, a thermal way densification and a chemical way hardening.

15. Use according to claim 14, wherein the chemical way hardening treatment of the thin film was a treatment by a base in a liquid or gas medium, preferably with ammonia vapours.

16. Use according to any of claims 1 to 15, wherein the gravimetric sensor is a quartz microbalance sensor.

17. Use according to any of claims 1 to 16, wherein the gravimetric sensor is a multisensor comprising several unit sensors at least one of which comprises a thin film of mesoporous silica as a sensitive material.

18. Use according to any of claims 1 to 17, wherein the nitro compound(s) to be detected or assayed is/are chosen from nitroaromatic compounds, nitramines, nitrosamines and nitric esters.

19. Use according to any of claims 1 to 18, wherein the nitro compound(s) to be detected or assayed is/are chosen from nitrobenzene, dinitrobenzene, tri-nitrobenzene, nitrotoluene, dinitrotoluene, trinitrotoluene, dinitrofluorobenzene, dinitrotrifluoromethoxybenzene, aminodinitrotoluene, dinitrotrifluoromethylbenzene, chlorodinitrotrifluoromethylbenzene, hexanitrostilbene, trinitrophenylmethylnitramine and trinitrophenol.

FIG. 1

FIG. 2

FIG. 3

FIG. 4

FIG. 5

FIG. 6

FIG. 7

## RÉFÉRENCES CITÉES DANS LA DESCRIPTION

**Documents brevets cités dans la description**

- FR 2703791 A

**Littérature non-brevet citée dans la description**

- **SHENGYANG et al.** *Colloid Polym. Sci.,* 2007, vol. 285, 721-728
- **SHENGYANG et al.** *ChemPhysChem,* 2006, vol. 7, 1902-1905
- **ROUQUÉROL et al.** *Pure & Applied Chemistry,* 1994, vol. 66 (8), 1739-1758
- **SANCHEZ-PEDRONO et al.** *Anal. Chem. Acta,* 1986, vol. 182, 285
- **PEDRONO et al.** *Anal. Chem. Acta,* 1986, vol. 182, 285